(19) Europäisches Patentamt
European Patent Office
Office européen des brevets

(11) Veröffentlichungsnummer: **0 014 381**
**A1**

# (12) EUROPÄISCHE PATENTANMELDUNG

(21) Anmeldenummer: **80100342.7**

(22) Anmeldetag: **23.01.80**

(51) Int. Cl.³: **C 07 F 9/40**
**A 01 N 57/20**

(30) Priorität: **31.01.79 DE 2903592**

(43) Veröffentlichungstag der Anmeldung:
**20.08.80 Patentblatt 80/17**

(84) Benannte Vertragsstaaten:
**AT BE CH DE FR GB IT NL**

(71) Anmelder: **BAYER AG**
**Zentralbereich Patente, Marken und Lizenzen**
**D-5090 Leverkusen 1, Bayerwerk(DE)**

(72) Erfinder: **Kühle, Engelbert, Dr.**
**von Bodelschwingh Strasse 42**
**D-5060 Bergisch Gladbach 2(DE)**

(72) Erfinder: **Hagemann, Hermann, Dr.**
**Roggendorfstrasse 55**
**D-5000 Koeln 80(DE)**

(72) Erfinder: **Behrenz, Wolfgang, Dr.**
**Untergruendemich 14**
**D-5063 Overath(DE)**

(72) Erfinder: **Hammann, Ingeborg, Dr.**
**Belfortstrasse 9**
**D-5000 Koeln(DE)**

(72) Erfinder: **Stendel, Wilhelm, Dr.**
**In den Birken 55**
**D-5600 Wuppertal 1(DE)**

## (54) Phosphorylierte Carbamoylverbindungen, Verfahren zu ihrer Herstellung und ihre Verwendung als Schädlingsbekämpfungsmittel.

(57) Die vorliegende Erfindung betrifft neue phosphorylierte Carbamoylverbindungen der allgemeinen Formeln

$$(R_1O)_2\underset{O}{\underset{\|}{P}}-CCl=CClO\underset{O}{\underset{\|}{C}}NH\underset{O}{\underset{\|}{C}}R_2 \qquad (Ia)$$

$$Cl_2C=\underset{O=P(OR_1)_2}{C}-OCONHCOR_2$$

in welcher $R^1$ und $R^2$ die in der Beschreibung angegebene Bedeutung besitzen.

Verbindungen der Formeln (Ia) und (Ib) erhält man durch Umsetzung von Tetrachlorethoxicarbamoylverbindungen der Formel

$$Cl_3C-CHClO\underset{O}{\underset{\|}{C}}NH\underset{O}{\underset{\|}{C}}R_2 \qquad (II)$$

mit einem Trialkylphosphit.

Die Verbindungen der Formeln Ia und Ib sind Schädlingsbekämpfungsmittel.

EP 0 014 381 A1

Croydon Printing Company Ltd.

0014381

BAYER AKTIENGESELLSCHAFT
Zentralbereich
Patente, Marken und Lizenzen

5090 Leverkusen, Bayerwerk
Ad-by -Kü
Typ Ia

# Phosphorylierte Carbamoylverbindungen, Verfahren zu ihrer Herstellung und ihre Verwendung als Schädlingsbekämpfungsmittel

Die vorliegende Erfindung betrifft neue phosphorylierte Carbamoyl-Verbindungen, ein Verfahren zu ihrer Herstellung sowie ihre Verwendung als Schädlingsbekämpfungsmittel, insbesondere als Insektizide.

Es ist bereits bekannt geworden, daß chlorhaltige organische Phosphorverbindungen eine insektizide Wirkung besitzen. So wird der [O,O-Dimethyl-(1-hydroxy-2,2,2-trichlorethyl)-phosphorsäureester] (Trichlorfon) seit vielen Jahren in der Landwirtschaft und auf dem Hygienesektor praktisch eingesetzt. Bezüglich der Wirkungsgeschwindigkeit befriedigt dieses Produkt nicht immer.

Es wurde nun gefunden, daß die neuen phosphorylierten Carbamoylverbindungen der allgemeinen Formeln

$$\underset{\overset{\|}{O}}{(R_1O)_2P}-CCl{=}CClO\underset{\overset{\|}{O}}{C}NH\underset{\overset{\|}{O}}{C}R_2 \qquad (Ia)$$

$$\underset{\overset{|}{O{=}P(OR_1)_2}}{Cl_2C{=}C}-OCONHCOR_2 \qquad (Ib)$$

in welchen

$R^1$ einen niederen Alkylrest, bevorzugt einen $C_1$-$C_4$-Alkylrest, und

$R^2$ einen gegebenenfalls substituierten Alkoxi-, Cycloalkoxi-, Aralkoxi-, Aryloxi-, Alkylmercapto-, Cycloalkylmercapto-, Aralkylmercapto-, Arylmercaptorest, den Ammoniakrest, einen aliphatischen, cycloaliphatischen, araliphatischen, aromatischen oder heterocyclischen Aminrest oder einen Oximinorest bedeuten, hohe Wirksamkeit als Schädlingsbekämpfungsmittel, insbesondere eine starke insektizide Eigenschaft aufweisen.

Weiter wurde gefunden, daß man die Verbindungen der Formeln (Ia) und (Ib) erhält, wenn man Tetrachlorethoxicarbamoylverbindungen der Formel

$$Cl_3C-CHClO\underset{\overset{\|}{O}}{C}NH\underset{\overset{\|}{O}}{C}R_2 \qquad (II)$$

in welcher

$R_2$ die oben angegebene Bedeutung hat, mit einem Trialkylphosphit der Formel

$$(R_1O)_3P \qquad (III)$$

in welcher

$R_1$ die oben angegebene Bedeutung hat, in Gegenwart eines Verdünnungsmittels umsetzt.

Es ist überraschend, daß die erfindungsgemäßen Verbindungen eine höhere und schneller eintretende Wirksamkeit als Schädlingsbekämpfungsmittel, insbesondere aber insektizide Aktivität besitzen als die zum Stand der Technik genannten Verbindungen. Die gefundenen neuen Verbindungen stellen somit eine Bereicherung der Technik dar.

**Der Reaktionsverlauf läßt sich bei Verwendung von Tetrachlorethoxicarbonylcarbamidsäuremethylester und Trimethylphosphit durch folgendes Formelschema wiedergeben:**

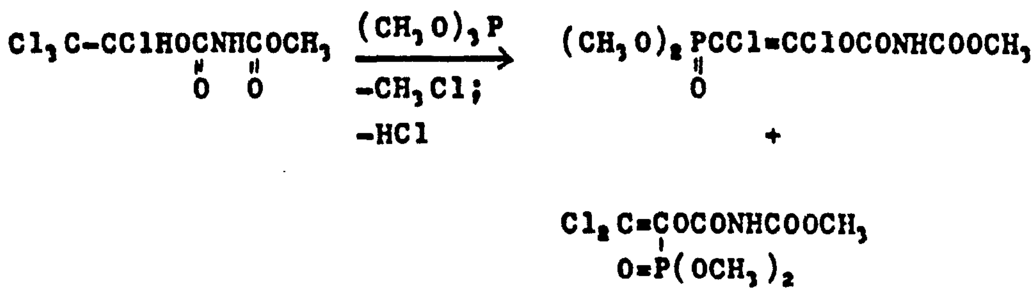


**Die als Ausgangsmaterial zu verwendenden Tetrachlorethoxicarbamoylverbindungen sind durch die Formel (II) definiert. In dieser Formel steht $R_2$ vorzugsweise für einen gesättigten oder ungesättigten Alkoxirest mit 1 - 6 C-Atomen, der gegebenenfalls durch Halogen, Alkoxi-, Alkylthio- oder Dialkylamino substituiert sein kann, einen gesättigten oder ungesättigten Cycloalkoxirest mit 5 - 7 C-Ringatomen, der gegebenenfalls durch Niederalkyl substituiert sein kann, einen Aralkoxirest mit 7 - 12 C-Atomen, der gegebenenfalls durch Halogen, Nitro, Alkyl, Alkoxi, Trihalogenmethyl substituiert sein kann, einen gegebenenfalls durch Halogen, Nitro, Alkyl, Alkoxi, Trihalogenmethyl substituierten Aryloxirest, einen gegebenen-**

falls durch Alkoxy, Alkylmercapto oder Dialkylamino substituierten Alkylmercapto, Cycloalkylmercapto oder Aralkylmercaptorest, einen gegebenenfalls durch Halogen, Nitro, Alkyl oder Trihalogenmethyl substituierten Arylmercaptorest, Ammoniak, einen primären oder sekundären aliphatischen, cycloaliphatischen, araliphatischen Aminrest mit 1 - 10 C-Atomen, einen gegebenenfalls durch Halogen, Nitro, Alkyl, Alkoxi, Trihalogenmethyl, Trihalogenmethoxi, Trihalogenmethylmercapto substituierten Arylaminrest, einen heterocyclischen Aminrest mit 1 - 3 Heteroatomen oder einen Oximinorest, der sich von aliphatischen, cycloaliphatischen, araliphatischen oder aromatischen Oximen ableitet.

Die Verbindungen der Formel II können durch Addition entsprechender Hydroxy-, Mercapto, Amino oder Oximinoverbindungen an das bekannte Tetrachlorethoxicarbonylisocyanat (Lit.: Zh. Org. Khim 12, 1963 (1976)) hergestellt werden.

Die zur Umsetzung ebenfalls benötigten Trialkylphosphite sind bekannt, wie z. B. Trimethyl-, Triethyl, Tripropyl- und Triisopropylphosphit.

Als Verdünnungsmittel kommen inerte organische Lösungsmittel in Frage. Hierzu gehören Ether wie Tetrahydrofuran und Dioxan; Kohlenwasserstoffe wie Benzol und Toluol; Chlorkohlenwasserstoffe wie Tetrachlorethan und Chlorbenzol.

Man führt die Umsetzung durch Erhitzen der Ausgangskomponenten im Molverhältnis 1 : 1 in einem Verdünnungsmittel bei 50 - 120°, vorzugsweise zwischen 80 - 120° durch, wobei Alkylchlorid und Chlorwasserstoff abgespalten werden. Nach beendigter Gasentwicklung ist die Reaktion vollständig.

Die Aufarbeitung der Reaktionslösung erfolgt durch Abdestillieren des Lösungsmittels, wobei die restlichen flüchtigen Anteile ebenfalls entfernt werden.

Die Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen.Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp.,Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp. Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp.,

Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..
Aus der Ordnung der Arachnida z.B. Scorpio maurus, Latrodectus mactans.
Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Zu den pflanzenparasitären Nematoden gehören Pratylenchus spp., Radopholus similis, Ditylenchus dipsaci, Tylenchulus semipenetrans, Heterodera spp., Meloidogyne spp., Aphelenchoides spp., Longidorus spp., Xiphinema spp., Trichodorus spp..

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff

und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyäthylen-Fettsäure-Ester, Polyoxyäthylen-Fettalkohol-Äther, z.B. Alkylarylpolyglykol-äther, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die Anwendung der erfindungsgemäßen Wirkstoffe erfolgt in Form ihrer handelsüblichen Formulierungen und/oder den aus diesen Formulierungen bereiteten Anwendungsformen.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gew.-% Wirkstoff, vorzugsweise zwischen 0,01 und 10 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

**Beispiel A**

$LT_{100}$-Test für Dipteren

Testtiere: Musca domestica resistent
Zahl der Testtiere: 25
Lösungsmittel: Aceton

2 Gewichtsteile Wirkstoff werden in 1000 Volumenteilen Lösungsmittel aufgenommen. Die so erhaltene Lösung wird mit weiterem Lösungsmittel auf die gewünschten geringeren Konzentrationen verdünnt.

2,5 ml Wirkstofflösung werden in eine Petrischale pipettiert. Auf dem Boden der Petrischale befindet sich ein Filterpapier mit einem Durchmesser von etwa 9,5 cm. Die Petrischale bleibt so lange offen stehen, bis das Lösungsmittel vollständig verdunstet ist. Je nach Konzentration der Wirkstofflösung ist die Menge Wirkstoff pro $m^2$ Filterpapier verschieden hoch. Anschließend gibt man die angegebene Anzahl der Testtiere in die Petrischale und bedeckt sie mit einem Glasdeckel.

Der Zustand der Testtiere wird laufend kontrolliert. Es wird diejenige Zeit ermittelt, welche für einen 100 %igen knock down-Effekt notwendig ist.

Bei diesem Test zeigen z.B. die folgenden Verbindungen der Herstellungsbeispiele überlegene Wirkung gegenüber dem Stand der Technik: 1-17, 23, 24.

Beispiel B

$LT_{100}$-Test für Dipteren

Testtiere: Aedes aegypti
Zahl der Testtiere: 25
Lösungsmittel: Aceton

2 Gewichtsteile Wirkstoff werden in 1000 Volumenteilen Lösungsmittel aufgenommen. Die so erhaltene Lösung wird mit weiterem Lösungsmittel auf die gewünschten geringeren Konzentrationen verdünnt.

2,5 ml Wirkstofflösung werden in eine Petrischale pipettiert. Auf dem Boden der Petrischale befindet sich ein Filterpapier mit einem Durchmesser von etwa 9,5 cm. Die Petrischale bleibt so lange offen stehen, bis das Lösungsmittel vollständig verdunstet ist. Je nach Konzentration der Wirkstofflösung ist die Menge Wirkstoff pro $m^2$ Filterpapier verschieden hoch. Anschließend gibt man die angegebene Anzahl der Testtiere in die Petrischale und bedeckt sie mit einem Glasdeckel.

Der Zustand der Testtiere wird laufend kontrolliert. Es wird diejenige Zeit ermittelt, welche für einen 100 %igen knock down-Effekt notwendig ist.

Bei diesem Test zeigen z.B. die folgenden Verbindungen der Herstellungsbeispiele überlegene Wirkung gegenüber dem Stand der Technik: 1-16, 23 , 24.

Beispiel C

$LD_{100}$-Test

Testtiere: Sitophilus granarius
Zahl der Testtiere: 25
Lösungsmittel: Aceton

2 Gewichtsteile Wirkstoff werden in 1000 Volumenteilen Lösungsmittel aufgenommen. Die so erhaltene Lösung wird mit weiterem Lösungsmittel auf die gewünschten Konzentrationen verdünnt.

2,5 ml Wirkstofflösung werden in eine Petrischale pipettiert. Auf dem Boden der Petrischale befindet sich ein Filterpapier mit einem Durchmesser von etwa 9,5 cm. Die Petrischale bleibt so lange offen stehen, bis das Lösungsmittel vollständig verdunstet ist. Je nach Konzentration der Wirkstofflösung ist die Menge Wirkstoff pro $m^2$ Filterpapier verschieden hoch. Anschließend gibt man die angegebene Anzahl der Testtiere in die Petrischale und bedeckt sie mit einem Glasdeckel.

Der Zustand der Testtiere wird 3 Tage nach Ansetzen der Versuche kontrolliert. Bestimmt wird die Abtötung in %. Dabei bedeutet 100 %, daß alle Testtiere abgetötet wurden; 0 % bedeutet, daß keine Testtiere abgetötet wurden.

Bei diesem Test zeigen z.B. die folgenden Verbindungen der Herstellungsbeispiele überlegene Wirkung gegenüber dem Stand der Technik: 1-17, 23, 24.

Beispiel D

Myzus-Test

Lösungsmittel: 3 Gewichtsteile Aceton
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea), die stark von der Pfirsichblattlaus (Myzus persicae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Blattläuse abgetötet wurden; 0 % bedeutet, daß keine Blattläuse abgetötet wurden.

Bei diesem Test zeigen z.B. die folgenden Verbindungen der Herstellungsbeispiele überlegene Wirksamkeit gegenüber dem Stand der Technik: 1,2,4,5,6,7,9,14,15,17, 24.

Beispiel E

Test mit Lucilia cuprina res.-Larven

Emulgator: 35 Gewichtsteile Äthylenglykolmonomethyläther
35 Gewichtsteile Nonylphenolpolyglykoläther

Zur Herstellung einer zweckmäßigen Wirkstoffzubereitung vermischt man drei Gewichtsteile Wirkstoff mit sieben Gewichtsteilen des oben angegebenen Lösungsmittel-Gemisches und verdünnt das so erhaltene Konzentrat mit Wasser auf die jeweils gewünschte Konzentration.

Etwa 20 Lucilia cuprina res.-Larven werden in ein Teströhrchen gebracht, welches ca. 1 $cm^3$ Pferdefleisch und 0,5 ml der Wirkstoffzubereitung enthält. Nach 24 Stunden wird der Abtötungsgrad bestimmt.

Bei diesem Test zeigen z.B. die folgenden Verbindungen der Herstellungsbeispiele überlegene Wirkung gegenüber dem Stand der Technik: 5

Beispiel F

Test mit Musca autumnalis

Lösungsmittel: 35 Gewichtsteile Äthylenglykolmonomethyläther
35 Gewichtsteile Nonylphenolpolylgkyoläther

Zur Herstellung einer zweckmäßigen Wirkstoffzubereitung vermischt man drei Gewichtsteile Wirkstoff mit sieben Gewichtsteilen des oben angegebenen Lösungsmittelgemisches und verdünnt das so erhaltene Konzentrat mit Wasser auf die jeweils gewünschte Konzentration.

10 adulte Musca autumnalis werden in Petrischalen gebracht, die Filterpapierscheiben entsprechender Größe enthalten, die einen Tag vor Versuchsbeginn mit 1 ml der zu testenden Wirkstoffzubereitung durchtränkt wurden. Nach 3 Stunden wird der Abtötungsgrad bestimmt.

Bei diesem Test zeigen z.B. die folgenden Verbindungen der Herstellungsbeispiele überlegene Wirkung gegenüber dem Stand der Technik: 2, 3, 4, 5 und 9.

Beispiele

Herstellung der Ausgangsprodukte der Formel II

$Cl_3C-CHClOCNHC-OCH_3$
(mit $\overset{\|}{O}$ unter den beiden C-Atomen: O O)

30 g Tetrachlorethoxicarbonylisocyanat werden in 100 ml Toluol gelöst und tropfenweise mit 5 g Methanol versetzt. Hierbei steigt die Temperatur bis 35° an. Man engt die Reaktionslösung i. V. ein und kristallisiert den Rückstand aus Waschbenzin um. F. 130 - 131°, Ausbeute 23 g = 68 % d. Th.

In analoger Weise erhält man:

$Cl_3-CCHClOCONHCOR_2$

| $R_2$ | Fp ($n_D^{20}$) | $R_2$ | Fp ($n_D^{20}$) |
|---|---|---|---|
| $-OC_2H_5$ | 88 - 89° | $-OCH_2CH_2OC_3H_7$-i | 90° |
| $-OC_3H_7$ i | 93 - 96° | | |
| $-OCH_2CH=CH_2$ | (1,5047) | 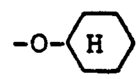 | 132-133° |
| $-OCH_2C\equiv CH$ | 133- 134° | 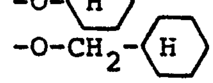 | 120-121° |
| $-OCH_2CH_2OCH_3$ | (1,4991) | | |
| $-OCH_2CH_2SC_2H_5$ | 98° | 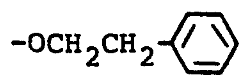 | 112-115° |
| 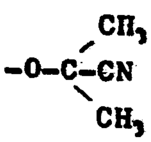 | 120° | 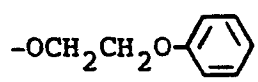 | helles Öl |
| 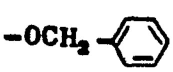 | 116 - 117° | 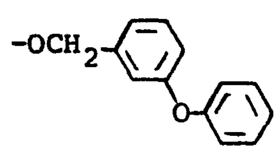 | (1.5700) |
| 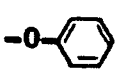 | 147 - 149° | | |
| 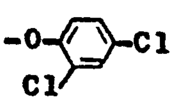 | 168° | | |

| $R_2$ | Fp ($n_D^{20}$) |
|---|---|
| 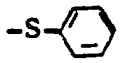 | 156 - 157° |
| $-NHC_4H_9$ t | 130° |
| 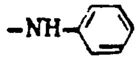 | 151 - 152° |
| 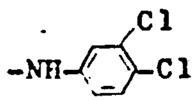 | 181° |
| 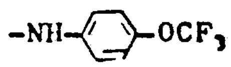 | 127 - 128° |
| $-ON=C(CH_3)SCH_3$ | 136 - 137° |

Beispiel 1:

$(CH_3O)_2P(=O)CCl=CClOCONHCOOCH_3$

\+ (1)

$Cl_2C=C(P(=O)(OCH_3)_2)OCONHCOOCH_3$

8 g N-(Tetrachlorethoxicarbonyl)carbamidsäuremethylester (1/30 Mol) werden in 70 ml Toluol gelöst und mit 5 g Trimethylphosphit versetzt. Man erhitzt die Reaktionslösung unter Rühren bis auf 100°. Bei dieser Temperatur spaltet sich Methylchlorid und Chlorwasserstoff ab. Man hält 1 Stde. lang auf dieser Temperatur und erhitzt kurze Zeit zum Rückfluß. Anschließend wird die Lösung i. V. eingeengt, wobei 9 g eines farblosen Oels zurückbleiben. $n_D^{20}$ 1,4682. Ausbeute quantitativ.

| | | | | |
|---|---|---|---|---|
| Ber.: | C 26,1% | H 3,1% | N 4,3% | Cl 22,0% |
| Gef.: | 25,3% | 3,1% | 3,9% | 22,4% |

In analoger Weise erhält man die Verbindungsgemische

$(CH_3O)_2\overset{O}{\overset{\|}{P}}CCl{=}CClOCONH\underset{\underset{O}{\|}}{C}{-}R_2 \quad + \quad Cl_2C{=}\underset{O{=}P(OCH_3)_2}{\underset{|}{C}}OCONHCOR_2$

| $R_2$ | | $n_D^{20}$ | $R_2$ | | $n_D^{20}$ |
|---|---|---|---|---|---|
| $-OC_2H_5$ | (2) | 1,4694 | $-OCH_2CH_2OC_3H_7$ i | (18) | 1.4665 |
| $-OC_3H_7$ i | (3) | 1,4611 | 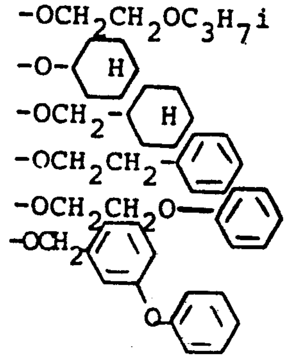 | (19) | 1.4858 |
| $-OCH_2CH{=}CH_2$ | (4) | 1,4807 | | (20) | 1.4845 |
| $-OCH_2C{\equiv}CH$ | (5) | 1,4813 | | (21) | 1.5148 |
| $-OCH_2CH_2OCH_3$ | (6) | 1,4712 | | (22) | 1.5214 |
| $-OCH_2CH_2SC_2H_5$ | (7) | 1,4827 | | (24) | 1.5396 |
| 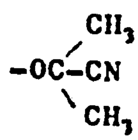 | (8) | 1,4831 | | | |
| 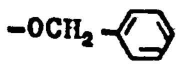 | (9) | 1,5119 | | | |
| 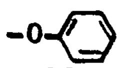 | (10) | | | | |
| 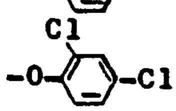 | (11) | 1,5240 | | | |
| 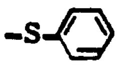 | (12) | 1,5430 | | | |
| $-NHC_4H_9$ t | (13) | 1,4802 | | | |
| 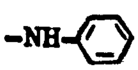 | (14) | 1,5225 | | | |
| 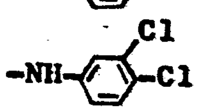 | (15) | 1,5535 | | | |
| 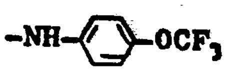 | (16) | 1,4997 | | | |
| 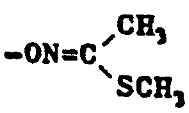 | (17) | 1,4912 | | | |

**Beispiel 2:**

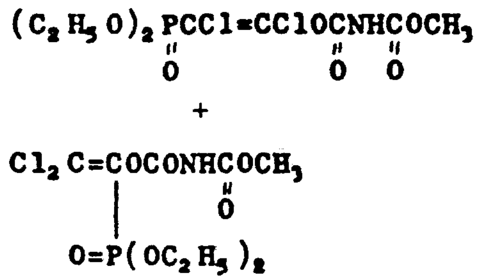

(23)

30 g N-(Tetrachlorethoxicarbonyl)carbamidsäuremethylester werden unter Zusatz von 19 g Triethylphosphit in 120 ml Toluol 5 Stdn. lang auf 100° erhitzt. Man engt i. V. ein und erhält als Rückstand 34 g = 93 % d. Th. eines hellgelben Oeles $n_D^{20}$: 1,4612.
Nach NMR-Spektrum und DC besteht das Reaktionsprodukt aus den beiden angegebenen Isomeren.

| | | | | |
|---|---|---|---|---|
| Ber.: | C 30,9% | H 4,0% | N 4,0% | Cl 20,3% |
| Gef.: | 30,9% | 4,6% | 4,0% | 19,7% |

Patentanspruch

1.) Phosphorylierte Carbamoylverbindungen der allgemeinen Formeln Ia und Ib

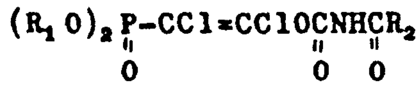

Ia

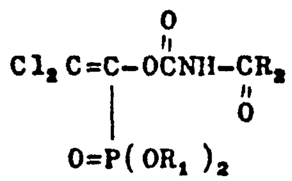

Ib

in welchen

$R_1$ einen niederen Alkylrest und

$R_2$ einen gegebenenfalls substituierten Alkoxi-, Cycloalkoxi, Aralkoxi, Aryloxi, Alkylmercapto, Cycloalkylmercapto, Aralkylmercapto, Arylmercaptorest, den Ammoniakrest, einen aliphatischen, cycloaliphatischen, araliphatischen, aromatischen, heterocyclischen Aminrest oder einen Oximinrest bedeuten.

2.) Verfahren zur Herstellung von phosphorylierten Carbamoylverbindungen der allgemeinen Formeln Ia und Ib, dadurch gekennzeichnet, daß man Tetrachlorethoxicarbamoylverbindungen der Formel

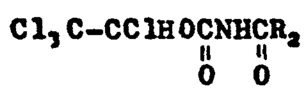

II

in welcher $R_2$ die oben angegebene Bedeutung hat, mit einem Trialkylphosphit der Formel

$$(R_1O)_3P \qquad III$$

in welcher $R_1$ die oben angegebene Bedeutung hat, in Gegenwart eines Verdünnungsmittels umsetzt.

3. Schädlingsbekämpfungsmittel, gekennzeichnet durch einen Gehalt an Verbindungen gemäß Anspruch 1.

4. Insektizide Mittel nach Anspruch 3, gekennzeichnet durch einen Gehalt an Verbindungen gemäß Anspruch 1.

5. Verfahren zur Bekämpfung von Schädlingen insbesondere von Insekten, dadurch gekennzeichnet, daß man Verbindungen gemäß Anspruch 1 auf die genannten Schädlinge bzw. deren Lebensraum einwirken läßt.

6. Verwendung von Verbindungen gemäß Anspruch 1 zur Bekämpfung von Schädlingen, insbesondere von Insekten.

7. Verfahren zur Herstellung von Schädlingsbekämpfungsmitteln, insbesondere von insektiziden Mitteln, dadurch gekennzeichnet, daß man Verbindungen gemäß Anspruch 1 mit Streckmitteln und/oder oberflächenaktiven Mitteln mischt.

0014381

Europäisches Patentamt

# EUROPÄISCHER RECHERCHENBERICHT

Nummer der Anmeldung
EP 80 10 0342

| EINSCHLÄGIGE DOKUMENTE | | | KLASSIFIKATION DER ANMELDUNG (Int.Cl.³) |
|---|---|---|---|
| Kategorie | Kennzeichnung des Dokuments mit Angabe, soweit erforderlich, der maßgeblichen Teile | betrifft Anspruch | |
| | DE - B - 1 078 370 (NORDDEUTSCHE AFFINERIE)<br>* Das ganze Dokument *<br>-- | 1,3 | C 07 F 9/40<br>A 01 N 57/20 |
| | DE - B - 1 193 044 (BAYER)<br>* Das ganze Dokument *<br>-- | 1,3 | |
| | FR - A -1 357 950 (BAYER)<br>* Das ganze Dokument *<br>-- | 1,3 | RECHERCHIERTE SACHGEBIETE (Int. Cl.³) |
| | DE - B - 1 212 967 (BAYER)<br>* Das ganze Dokument *<br>-- | 1,3 | C 07 F 9/40<br>9/38 |
| | US - A - 3 069 312 (G.K. KOHN)<br>* Das ganze Dokument *<br>-- | 1,3 | |
| | DE - A - 2 038 011 (CIBA AG)<br>* Seiten 26-38 *<br>---- | 1,3 | KATEGORIE DER GENANNTEN DOKUMENTE<br>X: von besonderer Bedeutung<br>A: technologischer Hintergrund<br>O: nichtschriftliche Offenbarung<br>P: Zwischenliteratur<br>T: der Erfindung zugrunde liegende Theorien oder Grundsätze<br>E: kollidierende Anmeldung<br>D: in der Anmeldung angeführtes Dokument<br>L: aus andern Gründen angeführtes Dokument |
| ⍉ | Der vorliegende Recherchenbericht wurde für alle Patentansprüche erstellt. | | &: Mitglied der gleichen Patentfamilie, übereinstimmendes Dokument |

| Recherchenort | Abschlußdatum der Recherche | Prüfer |
|---|---|---|
| Den Haag | 04-03-1980 | BESLIER |

EPA form 1503.1 06.78